# EUROPEAN PATENT SPECIFICATION

(11) **EP 0 858 770 B1**
(45) Date of publication and mention of the grant of the patent: **10.10.2001**
(21) Application number: 98102425.0
(22) Date of filing: 12.02.1998
(51) Int. Cl.: A61B 5/00

(54) **Device for thermographic analyses of selected organ portions and method for obtaining skin thermographic maps**
Vorrichtung zur Durchführung thermographischer Analysen von ausgewählten Teilen von Organen und Verfahren zur Gewinnung thermographischer Bilder der Haut
Appareil pour la réalisation d'analyses thermographiques de parties définies d'organes et procédé pour obtenir des cartes thermographiques de la peau

(30) Priority: 17.02.1997 CH 35397
(43) Date of publication of application: 19.08.1998
(73) Proprietor: Belfry Services Limited, Road Town, Tortola (VG)
(72) Inventor: Barton, Noel, Road Town, Tortola (VG)
(74) Representative: Modiano, Guido, Dr.-Ing.

(56) References cited:
- GB-A- 2 086 575
- US-A- 3 245 402
- US-A- 4 135 497
- US-A- 4 246 302
- US-A- 4 616 912

## Description

The present invention relates to a device for thermographic analyses of selected organ portions and to a method for obtaining skin thermographic maps.

Conventional thermographic methods and devices allow to detect the temperature of the body surface to be investigated. These devices often consist of flexible sheets which support liquid crystals (which change color as the temperature that they detect varies), which are meant to be rested against the surface of the skin of the body part to be investigated, to change color in relation to the detected temperature and to be in some cases photographed in order to provide a thermographic map of the body surface.

It has been noted that such thermographic investigations are highly dependent on skin temperature, which predominantly affects the detected values, to the point that the temperature readout often cannot be attributed correctly to the condition of the underlying organ. It has also been noted that it is possible to have reliable indications as to the health of the underlying organ if it is possible to assess the situation of the deep circulation of the blood vessels by means of a thermographic map, by trying to reduce the incidence of surface temperature. This is done for example for thyroid and breast investigations.

It has also been noted that reliable indications as to the health of an organ can be obtained by studying a set of photographs of the thermogram, taken at different and successive times.

US-A-4 616 812 discloses an apparatus for the photographic recording of plate thermography which comprises two parallel rails to whose rear end is mounted a frame for holding a thermographic plate and to whose front end is mounted a camera. A camera operating grip is mounted at the center of gravity at the rails, which grip is provided with electric cables for tripping the camera and flash units mounted at ends of a crossbar connected to the rails. Magnetically adhering symbol plates are connectable to the plate frame for identifying, for example, "right breast", "left breast", "after cooling with air blower", etc.

US-A-3 245 402 discloses an infrared thermography diagnosis method which includes pre-chilling of the skin with gradual warming up whereby thermographs are taken with the skin temperature too low to anything worthwhile and then one or more as the tissues and the skin warm up.

The aim of the present invention is to obviate the above-mentioned drawbacks of conventional methods and devices, i.e., to provide a method and a device for thermographic analyses of selected organ portions which allow to provide observations which are in no way affected by the skin temperature of the patient, so that findings can be attributed correctly to the situation of blood circulation in the underlying organs to be investigated.

Within the scope of this aim, another object of the present invention is to provide a method and a device for thermographic analyses of selected organ portions which allow to obtain images of thermographic conditions at successive time intervals, in order to allow functional evaluation of deep blood circulation so as to detect both phlogistic and preneoplastic conditions (which have a very high metabolic demand) and actual neoplasms (states related to a certain spot, which are isolated and therefore clearly evident).

Another object of the present invention is to provide a method and a device which are simple, relatively easy to provide in practice, safe in use, effective in operation and have a relatively low cost.

In accordance with the invention, there is provided a device for thermographic analyses and a method for obtaining a thermographic map of a skin region as defined in the appended claims 1 and 6.

Further characteristics and advantages will become apparent from the following detailed description of a preferred but not exclusive embodiment of a method and a device for thermographic analyses of selected organ portions according to the present invention, illustrated only by way of non-limitative example in the accompanying drawings, wherein:

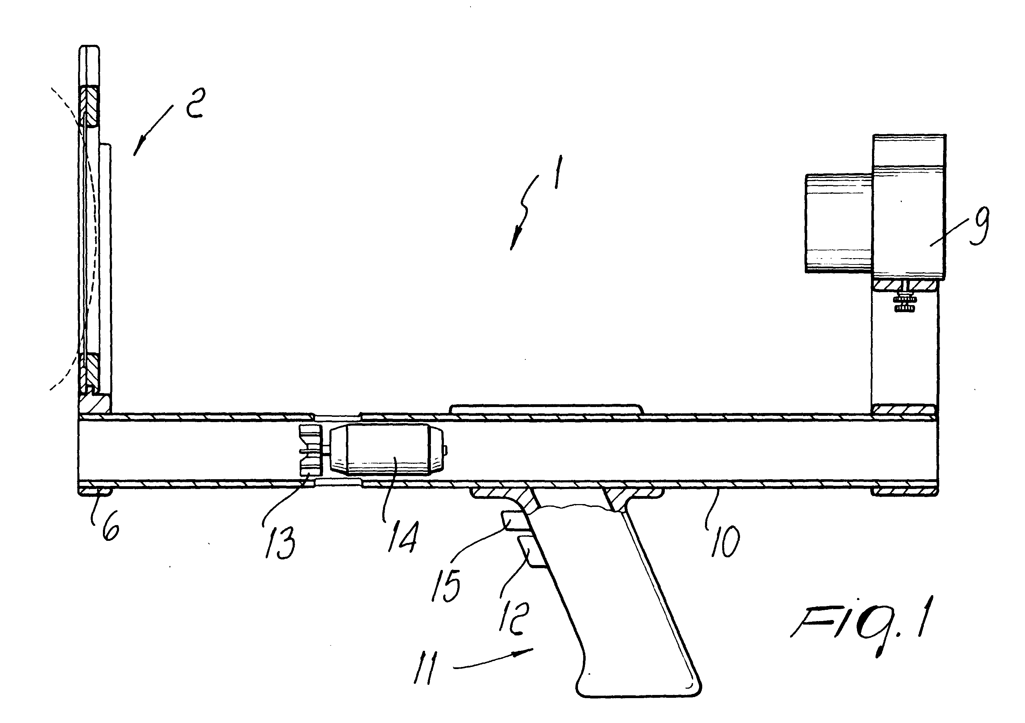

figure 1 is a sectional side view of a device for thermographic analyses of selected organ portions according to the present invention;

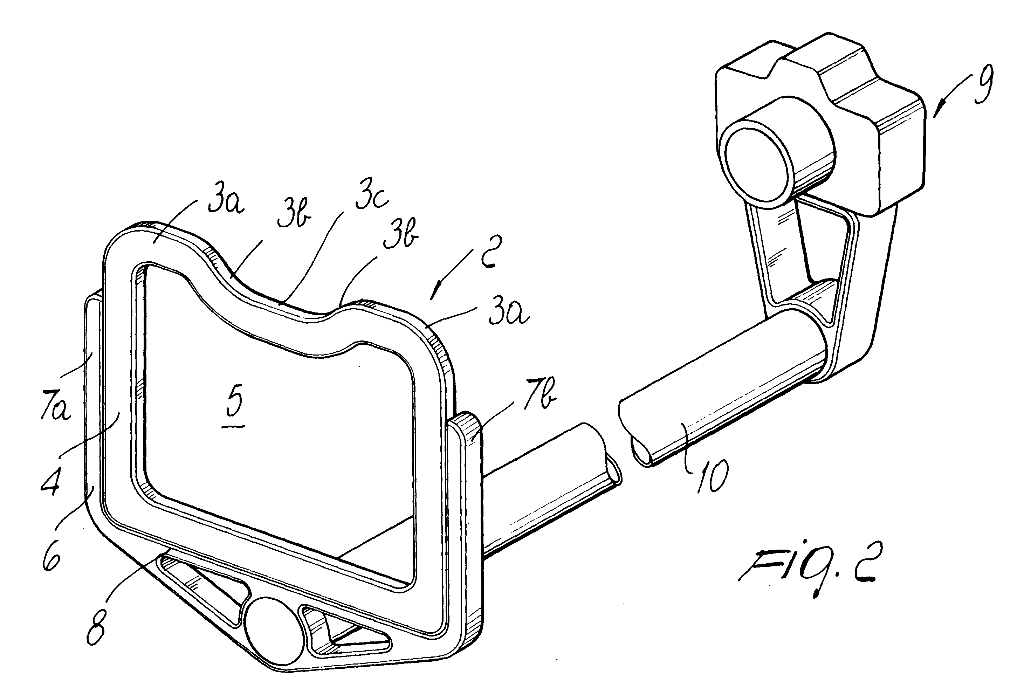

figure 2 is a perspective view of the device for thermographic analyses of selected organ portions according to the present invention;
figures 3A and 3B are schematic views of the sequence of operations related to the method for thermographic analyses of selected organ portions according to the invention.

With particular reference to the above figures, the reference numeral 1 generally designates a device for thermographic analyses of selected organ portions according to the invention.

Known thermographic analysis methods consist in resting against the skin of the region to be investigated a heat-sensitive deformable and flexible screen and, in some cases, in taking a photograph of said screen: the innovation that characterizes the invention is that the skin of the region against which the screen is pressed is cooled beforehand with a jet of air: the heat-sensitive screen rested on the cooled skin is therefore not affected, as occurs according to known methods, by surface temperature but is predominantly affected by the heat produced by the underlying blood circulation.

Since the circulatory flows towards a certain region are due to metabolic demands that are higher than those of surrounding regions, it is thus possible to detect both phlogistic states and preneoplastic states (which have a very high metabolic demand) and actual neoplasms (states related to a certain spot, which are isolated and accordingly very evident).

The device 1 comprises a thermographic screen 2 which is substantially rectangular, with corners and an. upper edge affected by respective radiused regions 3a, 3b which are shaped like a quarter-circle, are connected by a linear portion 3c, and allow to perform thermographic analysis of body regions which are not accessible with screens having sharp corners and a continuous edge, for example for the lateral examination of the breast, where it allows to investigate also upper regions below the axillary fossa, for a full analysis of the axillary extension of the mammary gland.

The screen 2 is constituted by a perimetric frame 4 made of a material such as plastics, inside which a thin film 5 is stretched. The film is made of a material such as cellophane, or of a material known by the trade-name Mylar, with a thickness between two and eight hundredths of a millimeter. A layer of black paint is applied to the film 5 at room temperature, and a solution of liquid crystals of the commercial type, with various grades of reactivity to heat according to the temperature at which the screen is to react, is applied to the surface. Accordingly, a plurality of screens of different sensitivity is available for selection at the time of analysis.

Advantageously, after applying the layer of black paint, the solution of liquid crystals is distributed on the film 5 in a plurality of successive layers, at least three, allowing each layer the time to dry before spreading the next layer: stratification of the liquid crystal solution should conveniently occur in no less than twenty minutes.

Preferably, screens with five types of sensitivity, corresponding to temperatures between 31 and 35 degrees Celsius, are provided.

The reference numeral 6 designates a supporting frame for the thermographic screen 2, which has two lateral guides 7a, 7b and a lower guide 8 which have a substantially U-shaped cross-section for the sliding insertion of the screen 2.

The reference numeral 9 designates a photographic camera, or an imaging machine of another kind but of the camera-like type, and the reference numeral 10 designates a spacer, at the opposite ends whereof the supporting frame 6 and the camera 9 are rigidly coupled. In a planned version, the spacer 10 is simply constituted by a thin-walled tube having a relatively large diameter: advantageously, in an intermediate position the spacer 10 has a pistol grip 11 with a trigger 12 for operating the camera, which is obviously orientated and adjusted appropriately so as to take color photographs of the screen 2.

The reference numeral 13 designates a fan for cooling the surface of the skin, with a motor assembly 14 which is powered by a battery or by the mains and is associated with the spacer 10. The cooling fan 13 and the corresponding motor assembly 14 can be accommodated inside the spacer 10 or be fixed outside the spacer. The fan 13 is activated by means of a trigger 15 which is also mounted in the pistol grip 11.

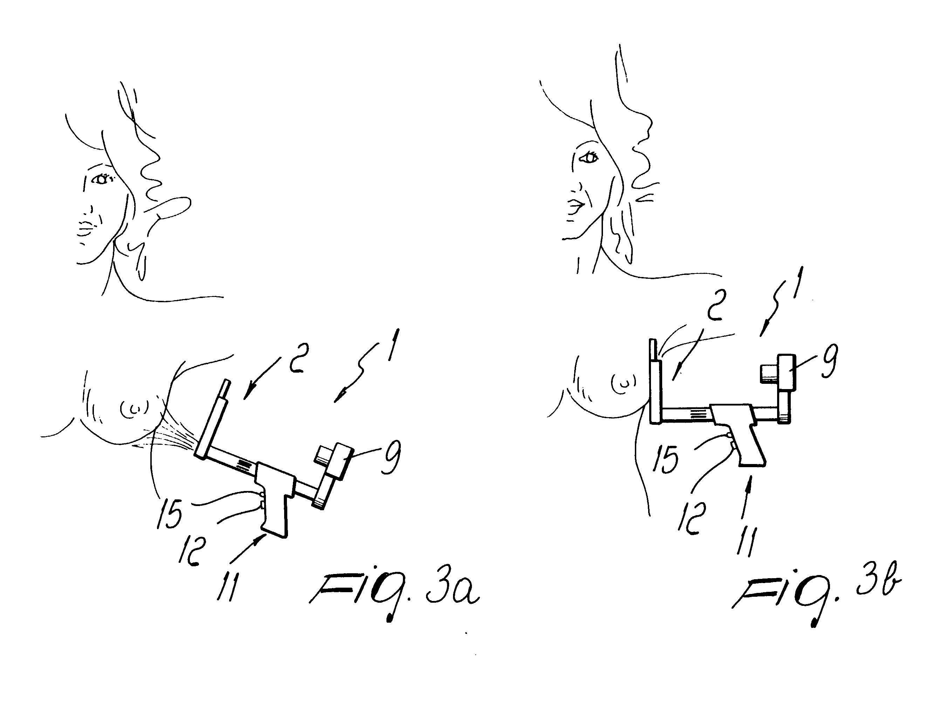

The fan 13 allows to cool the skin before resting the screen 2 on the patient and before taking the photographs. According to the method and by means of the device according to the present invention, a plurality of successive photographs are taken in order to provide a sequence of thermographic maps of the skin, starting from a low and uniform temperature condition. Figure 3 schematically illustrates the cooling step 3a and the image-taking step 3b for performing breast investigation.

It has been found that the study of successive maps allows to detect the flow of deep circulatory currents and to evaluate the health of internal organs.

It has thus been shown that the invention achieves the intended aim and objects.

The invention thus conceived is susceptible of numerous modifications and variations, all of which are within the scope of the inventive concept.

All the details may also be replaced with other technically equivalent elements.

In practice, the materials employed, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (1) for thermographic analyses of selected organ portions, comprising:
a thermographic screen (2);
a supporting frame (6) for said thermographic screen (2);
an imaging camera (9);
a spacer (10) at the opposite ends whereof said supporting frame (6) and said imaging camera (9) are rigidly coupled;
**characterized in that** said device (1) comprises a fan (13) for cooling the skin surface of selected organ portions, which is associated with said spacer (10) and is suitable to cool the skin before resting said screen (2) on the skin of the patient and taking at least one photograph.

2. A device (1) according to claim 1, **characterized in that** said thermographic screen (2) is substantially rectangular, with the upper comers and edge affected by respective radiused regions (3a,3b) shaped like a quarter-circle, which are suitable to allow analysis of body regions which cannot be accessed with screens having sharp corners and a continuous edge.

3. A device (1) according to any one or more of the preceding claims, **characterized in that** said spacer (10) is tubular and said cooling fan (13) and a corresponding motor assembly (14) are accommodated inside it.

4. A device (1) according to any one or more of the preceding claims, **characterized in that** said spacer (10) has a pistol grip (11) whereat triggers (12,15) are provided for actuating said fan (13) and said imaging camera (9).

5. A device (1) according to any one or more of the preceding claims, **characterized in that** said thermographic screen (2) comprises a thin supporting film (5) on which a layer of black paint is applied, at least three layers of a solution of liquid crystals being distributed on said layer of paint.

6. A method for obtaining a thermographic map of a skin region with a device (1) as defined in any of claims 1-5 which comprises the steps of resting against the skin region to be investigated with the screen (2) of said device which is a heat-sensitive deformable and flexible screen (2) and taking a photograph of said screen (2) with said camera (9) of said device (1), and comprising a first step of cooling said skin region with a jet of air by said fan (13) of said device (1) before resting said screen (2) thereon.

7. A method according to claim 6, said method comprising in succession the steps of activating said fan (13) to blow an air jet on said skin region, resting said thermographic screen (2) against said skin region and taking at least one photograph of said thermographic screen (2) into said imaging camera (9).

8. A device (1) according to any one or more of the preceding claims 1-5, **characterized in that** said thermographic screen (2) comprises a thin supporting film (5) having a thickness between two and eight hundredths of a millimeter.

9. A device (1) according to claim 8, **characterized in that** said film (5) is made of cellophane or of a material known by the trade-name Mylar.

## Patentansprüche

1. Vorrichtung (1) für thermographische Analysen von ausgewählten Organteilen, welche folgendes umfaßt:
einen thermographischen Schirm (2);
einen Tragrahmen (6) für den thermographischen Schirm (2);
eine Abbildungskamera (9);
einen Abstandhalter (10), an dessen entgegengesetzte Enden der Tragrahmen (6) und die Abbildungskamera (9) starr gekoppelt sind;
**dadurch gekennzeichnet, daß** die Vorrichtung (1) ein Gebläse (13) zum Kühlen der Hautoberfläche von ausgewählten Organteilen umfaßt, das zum Abstandhalter (10) gehört und zum Kühlen der Haut, bevor der Schirm (2) an die Haut des Patienten angelegt wird und mindestens eine Fotografie aufgenommen wird, geeignet ist.

2. Vorrichtung (1) nach Anspruch 1, **dadurch gekennzeichnet, daß** der thermographische Schirm (2) im wesentlichen rechteckig ist, wobei die oberen Ecken und die obere Kante mit jeweiligen abgerundeten Bereichen (3a, 3b), die wie ein Vierteilkreis geformt sind, versehen sind, welche geeignet sind, um eine Analyse von Körperbereichen zu ermöglichen, die mit Schirmen mit spitzen Ecken und einer durchgehenden Kante nicht zugänglich sind.

3. Vorrichtung (1) nach einem oder mehreren der vorangehenden Ansprüche, **dadurch gekennzeichnet, daß** der Abstandhalter (10) röhrenförmig ist und das Kühlgebläse (13) und eine entsprechende Motoranordnung (14) innerhalb diesem untergebracht sind.

4. Vorrichtung (1) nach einem oder mehreren der vorangehenden Ansprüche,
**dadurch gekennzeichnet, daß** der Abstandhalter (10) einen Pistolengriff (11) aufweist, an dem Auslöser (12, 15) zum Betätigen des Gebläses (13) und der Abbildungskamera (9) vorgesehen sind.

5. Vorrichtung (1) nach einem oder mehreren der vorangehenden Ansprüche, **dadurch gekennzeichnet, daß** der thermographische Schirm (2) eine dünne Trägerfolie (5) umfaßt, auf die eine Schicht von schwarzer Farbe aufgetragen ist, wobei mindestens drei Schichten einer Lösung von Flüssigkristallen auf der Farbschicht verteilt sind.

6. Verfahren zum Erhalten einer thermographischen Abbildung eines Hautbereichs mit einer Vorrichtung (1) nach einem der Ansprüche 1-5, welches die Schritte des Anlegens an den Hautbereich, der mit dem Schirm (2) der Vorrichtung untersucht werden soll, welcher ein wärmeempfindlicher, verformbarer und biegsamer Schirm (2) ist, und des Aufnehmens einer Fotografie des Schirms (2) mit der Kamera (9) der Vorrichtung (1) umfaßt, und einen ersten Schritt des Kühlens des Hautbereichs mit einem Luftstrahl durch das Gebläse (13) der Vorrichtung (1), bevor der Schirm (2) an diesen angelegt wird, umfaßt.

7. Verfahren nach Anspruch 6, wobei das Verfahren nacheinander die Schritte des Aktivierens des Gebläses (13), um einen Luftstrahl auf den Hautbereich zu blasen, des Anlegens des thermographischen Schirms (2) an den Hautbereich und des Aufnehmens von mindestens einer Fotografie des thermographischen Schirms (2) in der Abbildungskamera (9) umfaßt.

8. Vorrichtung (1) nach einem oder mehreren der vorangehenden Ansprüche 1-5, **dadurch gekennzeichnet, daß** der thermographische Schirm (2) eine dünne Trägerfolie (5) mit einer Dicke zwischen zwei- und achthundertstel Millimeter umfaßt.

9. Vorrichtung (1) nach Anspruch 8, **dadurch gekennzeichnet, daß** die Folie (5) aus Zellophan oder aus einem unter dem Handelsnamen Mylar bekannten Material besteht.

## Revendications

1. Dispositif (1) pour analyses thermographiques de portions d'un organe choisi, ledit dispositif comprenant :
un écran thermographique (2) ;
un cadre de support (6) pour ledit écran thermographique (2) ;
une caméra d'imagerie (9) ;
un espaceur (10) aux extrémités opposées duquel ledit cadre de support (6) et ladite caméra d'imagerie (9) sont rigidement couplés ;
ledit dispositif (1) étant **caractérisé en ce qu'**il comprend un ventilateur (13) pour refroidir la surface de la peau des portions de l'organe choisi, qui est associé avec ledit espaceur (10) et convient au refroidissement de la peau avant de placer ledit écran (2) sur la peau du patient et de prendre au moins un cliché.

2. Dispositif (1) suivant la revendication 1, **caractérisé en ce que** ledit écran thermographique (2) est essentiellement rectangulaire, avec des coins et arrête supérieurs présentant des régions respectives arrondies (3a, 3b) en forme approximative de quart de cercle qui conviennent pour permettre 1' analyse de régions corporelles auxquelles on ne peut pas accéder avec des écrans ayant des coins pointus et une arrête continue.

3. Dispositif (1) suivant l'une ou plusieurs des revendications précédentes, **caractérisé en ce que** ledit espaceur (10) est tubulaire et ledit ventilateur de refroidissement (13) et un ensemble moteur correspondant sont logés dans son intérieur.

4. Dispositif (1) suivant l'une ou plusieurs des revendications précédentes, **caractérisé en ce que** ledit espaceur (10) a une poignée en forme de crosse (11) sur laquelle sont montées des gâchettes (12, 15) de commande dudit ventilateur (13) et de ladite caméra d'imagerie (9).

5. Dispositif (1) suivant l'une ou plusieurs des revendications précédentes, **caractérisé en ce que** ledit écran thermographique (2) comprend un film support mince (5) sur lequel une couche de peinture noire est appliquée, au moins trois couches d'une solution de cristaux liquides étant réparties sur ladite couche de peinture.

6. Procédé pour obtenir une carte thermographique d'une région de la peau avec un dispositif tel que défini dans l'une quelconque des revendications 1 à 5, ledit procédé comprenant les étapes de mise en place contre la région de la peau à étudier de l'écran (2) dudit dispositif qui est un écran (2) sensible à la chaleur, déformable et flexible, et de prise d'un cliché dudit écran (2) avec ladite caméra (9) dudit dispositif (1), et comprenant une première étape de refroidissement de ladite région de peau avec un jet d'air au moyen dudit ventilateur (13) dudit dispositif (1) avant de placer ledit écran (2) sur ladite région.

7. Procédé suivant la revendication 6, ledit procédé comprenant successivement les étapes de mise en marche dudit ventilateur (13) pour souffler un jet d'air sur la région de la peau, de mise en place dudit écran thermographique (2) contre ladite région de la peau, et de prise d'au moins un cliché dudit écran thermographique (2) avec ladite caméra d'imagerie (9).

8. Dispositif (1) suivant l'une ou plusieurs des revendications précédentes 1 à 5, **caractérisé en ce que** ledit écran thermographique (2) comprend un film support mince (5) ayant une épaisseur comprise entre 0,02 et 0,08 mm.

9. Dispositif (1) suivant la revendication 8, **caractérisé en ce que** ledit film (5) est en cellophane ou en un matériau connu sous le nom de marque de Mylar.
